# EUROPEAN PATENT APPLICATION

(11) **EP 1 004 664 A1**
(43) Date of publication of application: **31.05.2000**
(21) Application number: 98402925.6
(22) Date of filing: 24.11.1998
(51) Int. Cl.: C12N 15/12, C12N 15/62, A61K 38/17, C07K 14/47

(54) **Compositions and methods using lactadherin or variants thereof**

(71) Applicant: INSTITUT NATIONAL DE LA SANTE ET DE LA RECHERCHE MEDICALE (INSERM), 75654 Paris Cédex 13 (FR); Institut Curie, 75248 Paris Cedex 05 (FR)
(72) Inventor: The designation of the inventor has not yet been filed
(74) Representative: Becker, Philippe

(57) **Abstract**

The present invention relates to novel methods and composition that can be used in the biological, experimental and medical areas. More particularly, the invention relates to compositions and methods using lactadherin or variants thereof, or a corresponding nucleic acid, for regulating (i.e., inducing, inhibiting, stimulating, etc.) or monitoring an immune response, more specifically a CTL response, as well as for the delivery of molecules to dendritic cells, in vitro, ex vivo or in vivo.

## Description

The present invention relates to novel methods and composition that can be used in the biological, experimental and medical areas. More particularly, the invention relates to compositions and methods for regulating (i.e., inducing, inhibiting, stimulating, etc.) or monitoring an immune response, more specifically a CTL response, as well as for the delivery of molecules to dendritic cells, in vitro, ex vivo or in vivo.

The invention also relates to novel proteins, nucleic acids and vectors, and their use in the above discussed areas, for instance for the cross-priming of antigens, the phagocytosis of molecules by dendritic cells, etc. The present invention also provides methods of screening of molecules capable of regulating the CTL response.

Antigen recognition by the immune system can involve different mechanisms. In particular, antigen presentation to the helper T lymphocytes requires processing of said antigens by antigen presenting cells, and their association with class II molecules of the MHC complex. This recognition essentially leads to the stimulation of specific clones of B lymphocytes, which then produce antibodies specific for the antigen.

Antigen presentation to cytotoxic T lymphocytes (CTLs), on the other hand, requires the phagocytosis of the antigen by professional cells (essentially the dendritic cells), which cells contain the appropriate MHC class I molecules and are capable of stimulating antigen-specific CTLs. This phenomenon, called "cross-presentation" of antigens or "cross-priming" of CTLs occurs for instance with viral or tumor antigens, as well as with antigens contained in apoptotic bodies (resulting from viral infection, tumors, cell degeneration, or normal cell turnover, etc.) for instance.

Cross presentation of antigens is therefore a critical pathway for the elimination of antigens that are associated with pathological conditions. The capacity to induce or facilitate the cross-priming of antigens in vivo or in vitro would represent a very powerful approach to the treatment of such pathological conditions.

As indicated above, cross-priming of antigens essentially requires the phagocytosis , by dendritic cells, of particulate antigens, i.e., antigens included in bodies comprising for instance lipids, such as phosphatidyl serine. Such particulate antigens include for instance apoptotic bodies, vesicles or cell membranes. The invention now discloses the identification of a compound involved in the phagocytosis of particulate antigens by dendritic cells. More particularly, the invention discloses the identification of a compound that mediates the interaction between particulate antigens and dendritic cells, and functions as an adaptor of cross-priming.

The invention also describes novel compositions comprising a molecule associated with the above-identified adaptor or a variant thereof.

The invention can be used to regulate the immune response, in particular to reduce the immune response or, on the contrary, to facilitate (enhance) an immune response against a desired antigen. The invention also relates to novel vectors (such as proteins or liposomes) that can be used to deliver a selected molecule to a dendritic cell. The invention also relates to methods of screening of compounds that can regulate the cross-priming of antigens or the delivery of antigens to dendritic cells, as well as to methods of monitoring the immune response in a subject.

Antigen presenting cells (APCs) contain compartments of the endocytic pathway, including multivesicular bodies (MVBs), which are sites of loading of antigenic peptides on Major Histocompatibility Complex (MHC) class II molecule. These complexes are recognized by the T lymphocytes. In particular, EBV-transformed B-lymphocytes (B-EBV) have been shown to secrete such vesicles called exosomes (Raposo et al., Mol. Biol. Cell 8 (1997) 2619). B-EBV-derived exosomes contain peptide-MHC class II complexes, and are able to present them directly to CD4+ T lymphocytes (Raposo et al., 1997). More recently, vesicules secreted by another antigen presenting cells, dendritic cells (Dcs), have been described (Zitvogel et al., Nature Medicine 4 (1998) 594). DCs are the only APCs able to induce primary and secondary immune responses. To do so, they have developed a remarkable array of specific attributes, which makes them a unique APC. DCs exist under two maturation states: immature DCs are efficient for antigen endocytosis and phagocytosis, but inefficient for T cell activation, whereas mature DCs down regulate their internalization capacities, but express high levels of MHC and co-stimulation molecules, which makes them extremely efficient for T cell activation.

DCs-derived exosomes accumulate MHC class II and also bear MHC class I molecules. Exosomes produced by DCs exposed to tumor-derived antigenic peptides induce potent immune responses, leading to the rejection of established tumors (Zitvogel et al., 1998). Although the mechanisms of this spectacular anti-tumor effects are still unknown, the potency of exosome-mediated immune stimulation suggests that these vesicles may indeed mediate activation of T cell responses physiologically.

In an attempt to understand the molecular bases of the effects of DCs-derived exosomes on the immune system, the Applicants have determined the conditions of their production by DCs, and analyzed their protein composition. The results obtained show that exosomes secretion is down modulated upon DC maturation, and that exosomes concentrate a limited number of proteins. In particular, besides MHC class II molecules, one major adhesion and targeting membrane protein has been identified, which behaves as the molecular effector of the effects of exosomes on the immune system, and functions to address exosomes to a cellular target, in vivo.

More particularly, the invention now demonstrates that lactadherin (also known as MFG-E8) is produced by dendritic cells, exposed at the surface of exosomes, and is involved in the phagocytosis of particulate antigens by dendritic cells. The invention also discloses variants of lactadherin, that behaves as competitors of lactadherin and can be used to inhibit the phagocytosis of particulate antigens by dendritic cells. The invention also provides novel methods of delivering of molecules to dendritic cells, using vectors derived from lactadherin or variants thereof.

Mature human lactadherin is a 364 amino acid protein. Furthermore, lactadherin is a secreted protein and comprises a secretion signal. SEQ ID NO:1 represents the amino acid sequence of human lactadherin. Residues 1 to 23 (underlined) represent the secretion signal, and residues 24-387 represent the primary structure of the mature, secreted protein. The murine, bovine and porcine lactadherin have also been identified, isolated and sequenced (see for instance Stubbs et al., PNAS 87 (1990) 8417 ; Hvarregaard et al., Eur. J. Biochem. 240 (1996) 628). The sequence of the murine lactadherin is also represented on SEQ ID NO:2. Amino acid residues 1-22 represent the secretion signal, and residues 23-426 represent the primary structure of the mature, secreted protein.

Lactadherin has been originally described at the surface of milk fat globules (see Stubbs et al., supra). Also, this protein has been found so far essentially in mammary tissue and in cultured mammary epithelial cells. Except for their presence during lactogenesis, no function had been reported in the literature for lactadherins.

The invention now provides evidence that lactadherin is selectively produced by dendritic cells, more particularly immature dendritic cells, exposed at the surface of antigen vesicles and functions as an adaptor in antigens cross-presentation to CTLs. The invention therefore provides compositions and methods of use of lactadherin or variants thereof in the mediation of an immune response. The invention also discloses variants of lactadherin, which can be used to inhibit the cross priming, or to deliver antigens to antigen presenting cells, in particular to dendritic cells. The invention also discloses compositions comprising lactadherin or variants thereof in association with selected molecules, in particular hybrid molecules and/or liposome compositions comprising recombinant lactadherin molecules.

More particularly, it is an object of the present invention to use lactadherin or a variant thereof for the regulation of an immune response in vitro, ex vivo or in vivo. It is a particular object of this invention to use lactadherin or a variant thereof for the preparation of a composition for regulating an immune response, in particular a CTL response. It is another particular object of this invention to use lactadherin or a variant thereof for the inhibition of the cross-priming of antigens, in particular of the phagocytosis of antigens by dendritic cells.

Lactadherin comprises several functional domains. In particular lactadherin comprises two EGF-like domains, with a RGD sequence, which is involved in integrin binding. Integrin (and more specifically integrin α v β 5) being present at the surface of dendritic cells, this region of lactadherin is involved in the interaction of this protein with dendritic cells. This interaction furthermore appears to be selective. Lactadherin also comprises a domain homologous with the C-terminal region of human coagulation factors VIII and V, which represents a binding site for phospholipids, and in particular, phosphatidyl serine.

The invention now postulates and discloses that lactadherin interacts with particulate antigens, through the phospholipid binding sites, and with dendritic cells, through the RGD domain, and therefore mediates the phagocytosis of said particular antigens.

It is an object of the present invention to use lactadherin, or variants thereof, more particularly human lactadherin or variants thereof, to produce cytotoxic T lymphocytes specific for a selected antigen.

It is another object of the present invention to use lactadherin or variants thereof, more particularly human lactadherin or variants thereof, to deliver an antigen to dendritic cells.

Within the context of the present invention, lactadherin means a protein of mammalian origin, expressed at the surface of milk fat globules, and comprising a RGD site and a domain homologous to the factor VIII C-terminal region (i.e., a factor VIII-like domain). More specifically, the protein is of human, bovine, murine or porcine origin, more preferably of human origin. In a particular embodiment, lactadherin means human lactadherin having the sequence of SEQ ID NO: 1 or any functional analogs thereof. The term functional analogs designates natural analogs, resulting for instance from the polymorphism or from post-translational modifications, in particular from splicing(s) or single amino acid deletion, substitution or addition, with reference to SEQ ID NO: 1, and which retain at least one functional property of human lactadherin. Functional analogs also include lactadherin of other species, in particular murine lactadherin of SEQ ID NO:2.

Lactadherin used in the present invention can be either isolated from natural sources, or produced by recombinant DNA technology, as will be described below, for instance.

A "variant" of lactadherin, within the context of the present invention, means an artificial protein, i.e., a protein artificially created, and not found as such nature. Such a variant generally retains at least one biological property of lactadherin, for instance a binding property of lactadherin, and usually comprises one or several of the following alterations of the primary structure:
(a) a deletion of one or several amino acid residues, more preferably several contiguous amino acid residues. In a preferred embodiment, a deletion variant comprises a deletion of several amino acid residues, which overlap at least in part with a domain of lactadherin involved in the binding of phopholipids (PL), in particular phosphatidyl serine (PS), and/or integrin. The deletion can comprise up to 250 amino acids, preferably up to 200 amino acids, for instance. Furthermore, several deletions can be performed in one variant;
(b) a mutation in one or several amino acid residues, more preferably in at least one amino acid residue involved in the PS or integrin binding site of lactadherin. The mutation(s) can be conservative or non-conservative. Preferably, the mutation(s) in the binding site render(s) said binding site non-functional. More preferably, a variant of the invention comprises between 0 to 10 point mutations, even more preferably, 0 to 5;
(c) an addition of one or several amino acid residues. The added residues are preferably located on the N- or C- terminal end of the variant, although internal residues may also be added. The added residues can have different functions. For instance, they can represent tags that can be used in a purification method (GST, myc, etc.). The added residues can also be adaptors, that can be used for the association of the variant to a selected molecule such as an antigen, a nucleic acid, a receptor or a membrane lipid. Such adaptors can be for instance polylysine, other basic amino acids, a spacer, or any other DNA binding motif. The adaptor can also be a trafficking signal, such as a transmembrane domain of a protein or a lipid, for instance. Furthermore, the added residues can also represent a selected molecule, fused to the lactadherin, as will be discussed in more details below.

Also, as indicated above, variants according to this invention can combine one or several modifications of one or several of the above types.

A particular variant of lactadherin of this invention is a variant lacking a functional PL binding site (in particular a functional PS binding site) and/or a functional integrin binding site.

Variants lacking a functional PL binding site are essentially incapable of efficiently binding phospholipids such as phosphatidyl serine. Such variants however, may retain a functional integrin binding site and thus behave as competitors of natural lactadherin. Variants lacking a functional PL (i.e., phosphatidyl serine) binding site can be obtained by different methods. In a particular embodiment, said variants lack all or part of the phosphatidyl serine (PS) binding site. In another particular embodiment, such variants comprise a mutated PS binding site. Of course, variants of this invention can also comprise both deletion and mutation in the PS binding site. The phosphatidyl serine binding site of lactadherin essentially lies in the C-terminal amino acid residues of lactadherin, more particularly in the 150 C-terminal amino acid residues. A variant according to this invention is therefore any variant of lactadherin having a mutation and/or a deletion in/of the 150 C-terminal amino acid residues, and essentially incapable of binding phosphatidyl serine. A more preferred variant is a polypeptide comprising the sequence of SEQ ID NO:1, said sequence further comprising a mutation and/or a deletion in any one of amino acid residues 242 to 387.

In a particular embodiment of this invention, the variant comprises a deletion of at least 5, preferably at least 10, even more preferably at least 20 contiguous amino acids of the above C-terminal region. In a particular embodiment, the deletion encompasses the entire 150 C-terminal amino acid residues, more preferably the amino acid residues 242 to 387 of SEQ ID NO:1. Another specific embodiment of this invention is a lactadherin lacking the 25 C-terminal amino acids, in particular a polypeptide comprising amino acid residues 24-363 of SEQ ID NO:1 or a polypeptide comprising amino acid residues 23-402 of SEQ ID NO:2.

Examples of mutations include any suppression or substitution of one or several single amino acid residues in the 150 C-terminal region of lactadherin. The substitution can be either conservative or non-conservative, preferably non-conservative.

Particular examples of mutated variants of this invention include any lactadherin having one or several point mutation(s) in the 25 C-terminal amino acids, and essentially incapable of binding PS. More particular examples are polypeptides of SEQ ID NO:1 or 2, having one or several point mutation(s) in the 25 C-terminal amino acids.

These variants of the present invention are particularly interesting since they are capable of binding integrin αvβ5, i.e., dendritic cells, more preferably immature (human) dendritic cells, while they are essentially incapable of binding PS. These variants are particularly suited for the delivery of molecules to dendritic cells, in particular for the delivery of antigenic molecules to dendritic cells. These variants can also facilitate the cross-presentation of a selected antigen by dendritic cells, and thus the development of a specific CTL response against a selected antigenic molecule.

Variants of lactadherin lacking a functional integrin binding site are essentially incapable of binding efficiently dendritic cells. Such polypeptides can be any variant of lactadherin lacking all or part of the integrin binding site or which comprise a mutated integrin binding site. The integrin binding site of lactadherin essentially resides in an RGD motif located in the N-terminal part of the molecule. For instance, the integrin binding site of human lactadherin of SEQ ID NO:1 lies in residues 46-48 and the integrin binding site of murine lactadherin of SEQ ID NO:2 lies in residues 87-89. The invention therefore resides in any lactadherin comprising a mutation in and/or a deletion in or of the RGD motif, and which is essentially incapable of binding dendritic cells. Particular examples of such variants are polypeptides of SEQ ID NO:1 which lack one, two or all of the amino acid residues RGD at position 46-48, and polypeptides of SEQ ID NO:2 which lack one, two or all of the amino acid residues RGD at position 87-89.

Such variants can be used for instance to interfere with the cross-priming of antigens, in vitro, ex vivo or in vivo, by competing with naturally occurring lactadherin for the binding to particulate antigens. Other types of inhibitors are represented by polypeptides comprising essentially the phospholipid binding site of lactadherin, for instance peptides comprising essentially the amino acid residues 364-385 of SEQ ID NO:1, or variants thereof. Other inhibitors are represented by antibodies specific for lactadherin, in particular for lactadherin PL or integrin binding sites. Such antibodies (either polyclonals or monoclonals) can be raised by immunisation of an animal using corresponding peptides, and used to reduce an immune reaction.

Accordingly, an object of the present invention resides also in a variant of lactadherin which lacks a functional PL or integrin binding site.

The above variants can be prepared according to conventional techniques, known to the skilled artisan, and disclosed in more details in the Examples. For instance, they can be produced by recombinant DNA techniques, starting from a nucleic acid molecule encoding lactadherin. More particularly, a nucleic acid construct encoding lactadherin as defined above can be provided and modified according to conventional techniques to introduce mutation(s) and/or deletion(s) therein. Such techniques include digestion with restriction enzymes and/or exonucleases, ligation, site-directed mutagenesis, amplification, artificial nucleotide synthesis and the like. Such modifications can be performed in vitro, on isolated nucleic acid, generally produced or replicated in a competent host cell, such as eukaryotic or prokaryotic cells, or obtained by amplification with specific primers from a dendritic cell, for instance. In this regard, the invention also relates to a nucleic acid encoding a polypeptide as defined above. Said nucleic acid can be a DNA or a RNA molecule, or a synthetic nucleic acid. The invention also relates to any vector comprising a nucleic acid as defined above. Such vectors can be plasmids, cosmids, episomes, YACs, viral vectors, etc. Preferred vectors also comprise a promoter region that causes expression of the nucleic acid, as well as, optionally, a transcription termination signal (i.e., a polyadenylation sequence). The construction of such vectors and the choice of the promoter region can be made by the skilled artisan according to conventional techniques and routine experimentation. In particular, the vector can be a plasmid derived from pUC, pIC, pBR or pBS, or a viral vector prepared from retroviruses, adenoviruses or AAV, for instance. The invention also relates to a cell comprising a nucleic acid or a vector as defined above, and to their use, for instance in the production of variants as described above. The recombinant cell of the present invention can be prokaryotic (such as a bacteria) or eukaryotic, such as a yeast cell, a mammalian cell, an insect cell, etc. Said cells can be prepared by conventional techniques (electroporation, precipitation, gene gun, lipid-mediated transfection, etc.).

Another object of this invention lies in a composition comprising a variant of lactadherin which lacks a functional PL or integrin binding site. Such a composition can be used for instance to modulate the cross-priming of antigens.

A particular object of this invention lies in a composition comprising lactadherin or a variant thereof in association with a selected molecule.

Indeed, the invention also lies in the use of lactadherin or variants to deliver molecules to dendritic cells.

In this respect, any selected molecule can be associated to lactadherin or a variant thereof as disclosed above, in order to deliver said molecule to dendritic cells in vitro, ex vivo or in vivo, and/or to induce a CTL response. The selected molecule can be associated to lactadherin or a variant thereof in different manner, covalently or non covalently. Covalent association (binding) can be obtained either by chemical, enzymatic or genetic methods. In particular, chemical coupling can be made using conventional chemical adaptors and/or reactions, in particular by amide, amine, or acid bounds, with or without spacers such as cabonyldiimidazole, etc.

Chemical coupling can also be obtained using the streptavidin / biotin system or any other analogous method.

One particular method of coupling the selected molecule to the lactadherin involves recombinant DNA constructs (i.e., genetic coupling). In this regard, a chimeric nucleic acid can be prepared comprising a region encoding lactadherin or the variant thereof and a region encoding the selected polypeptide, operably linked together, i.e., in the same reading frame, so that, upon expression in a host cell, a fusion (chimeric) protein is obtained. The selected polypeptide (or peptide) can be fused to lactadherin or a variant thereof in N-terminal or C-terminal position. Preferably, the selected polypeptide or peptide (i.e., the heterologous moiety) is fused at the C-terminal end of lactadherin or a variant thereof according to this invention.

Accordingly, a particular object of the present invention resides in a chimeric protein (or polypeptide) comprising lactadherin or a variant thereof fused to a heterologous polypeptide or moiety.

Another particular object of the present invention lies in a product comprising lactadherin or a variant thereof coupled to a heterologous molecule. As indicated above, coupling can be covalent or non covalent.

Also, since lactadherin mediates the phagocytosis of particulate antigens, the invention also discloses artificial liposomes (or lipid vesicules) comprising lactadherin (preferably recombinant) or a variant thereof as disclosed above.

Lactadherin can be bound to the surface of the liposome, through its PL binding site. In this embodiment, the artificial liposome of this invention preferably comprises at least phosphatidyl serine as a constituent lipid. Lactadherin or the variant thereof can also be bound to the liposome membrane through a receptor moiety, capable of binding a ligand moiety present in the membrane of the liposome. In particular, as indicated above, a variant of lactadherin can be prepared comprising added residues or structure, which provide for the binding of said lactadherin variant to a selected receptor or ligand. In a particular embodiment, the variant of lactadherin comprises a peptide which binds a receptor present in the liposome membrane. The peptide can be biotin, which binds to streptavidin introduced in the liposome membrane.

In another particular embodiment, lactadherin or the variant is enclosed in the membrane of the liposome. The insertion (anchoring) can be achieved in particular by using a variant of lactadherin comprising an anchoring region, such as for instance a transmembrane domain. Such an anchoring region can be introduced in the variant as added residues in addition or in replacement of residues of lactadherin, and in particular in replacement of the PL binding site. The insertion can also be achieved by using a variant of lactadherin comprising a lipid at one of its extremities, preferably its C-terminal end, according to methods known to the skilled artisan. In this specific embodiment, a lipid (e.g. a fatty acid) is bound to the C-terminal end of lactadherin or a variant thereof having the capacity to bind integrin, using conventional method, and the resulting product is contacted with the liposome.

Thus, the invention also relates to any lipid vesicle composition comprising a recombinant protein comprising at least the integrin binding site of lactadherin, more preferably of human lactadherin. The lipid vesicle can be prepared according to known techniques. For instance, the selected lipids are contacted together in the appropriate medium to form lipid bilayers and vesicules. Lactadherin or the variant thereof can be added to the product before, during or after formation of the artificial liposomes. Also, an artificial liposome includes an exosome produced by a cell, which comprises a recombinant lactadherin molecule.

The invention also relates to a polypeptide comprising (a) a lactadherin amino acid sequence lacking a functional PL binding site and (b) a heterologous moiety allowing anchoring of said polypeptide in a lipid membrane.

The liposome compositions of this invention advantageously further comprise at least one selected molecule, such as an antigen. The selected molecule can be a lipid, polypeptide, nucleic acid, chemical molecule, etc.

Preferably, the selected molecule is a polypeptide (such as any protein, peptide, etc.) or a nucleic acid.

In this regard, the invention also relates to a polypeptide comprising (a) a lactadherin amino acid sequence lacking a functional PL binding site and (b) a heterologous moiety allowing binding of said polypeptide to a nucleic acid. Such a polypeptide is able to form complexes with nucleic acids, such as plasmids, for instance, and to target said nucleic acids to dendritic cells in vitro, ex vivo or in vivo. This invention also relates to a nucleic acid encoding the primary structure of the above polypeptide.

Even more preferably, the molecule is an antigen, i.e., a compound against which an immune response is sought, particularly a CTL response, a cytokine (such as IL12, IL4, GM-CSF, etc., which can stimulate the activation and/or differentiation of dendritic cells, for instance) or a toxic molecule, which may be used to eliminate dendritic cells.

Examples of antigen molecules comprise for instance viral proteins or peptides, bacterial proteins or peptides, or tumor antigens, such as MART-1, MAGE, BAGE, PSA, p53, Rb, Ras, etc. The antigen can be intact (the entire proteins) or in the form of a fragment thereof.

A preferred embodiment of the present invention is an immunogenic composition comprising lactadherin or a variant thereof in association with a selected antigenic molecule. Vaccines or immunogenic compositions of this invention can be used in any mammal, such as human beings or animals.

In this regard, the invention also relates to a hybrid polypeptide comprising (a) a lactadherin amino acid sequence lacking a functional PL binding site fused to (b) an antigenic polypeptide or peptide. The invention also relates to any nucleic acid encoding such a hybrid polypeptide. Said polypeptides and nucleic acids can be used to prepare vaccine compositions, in particular anti-tumor vaccine compositions, which can be administered in naked form, for instance by intramuscular injection.

The selected molecule can be present in the cytosol of the liposome and/or anchored into its membrane and/or bound to the surface of said liposome. The selected molecule can be combined with the liposome according to conventional methods as disclosed above, in particular by incubation of the molecule with the constituent lipids, prior to or during formation of the liposome.

The invention also relates to a recombinant host cell, in particular a recombinant bacteria, expressing a lactadherin or variant thereof. More preferably, the recombinant bacteria expresses lactadherin or a variant thereof having the capacity to bind integrin. The introduction of recombinant lactadherin or variant in bacteria, for instance, allows the uptake of bacterial membrane fractions by dendritic cells, and the efficient generation of immune responses against bacterial antigens, for instance.

The compositions, variants, chimeric proteins or liposomes of this invention can be used in vitro, ex vivo or in vivo. In vitro, they can be used to pulse or sensitize dendritic cells to a desired molecule, for instance. In this respect, the invention also discloses a method of delivering a molecule to a dendritic cell, preferably an immature dendritic cell, in vitro, ex vivo or in vivo, comprising contacting said dendritic cell (or a population of cells comprising a dendritic cell) with a compound, composition or chimeric protein as defined above. This method can be performed in any appropriate device (plate, dish, tubes, flask, etc.) with concentration of lactadherin or a variant thereof ranging from 0,01 to 1000 µg/ml, for instance.

The variants of lactadherin can also be used to target any compound to dendritic cells, in vitro, ex vivo or in vivo. For instance, the above disclosed variants of lactadherin having a functional integrin binding site can be used to target a vector (such as a viral, a polymeric, a cationic or a polypeptide vector) to dendritic cells. Particular examples include a recombinant adenovirus. Therefore, the invention also relates to the use of lactadherin or a variant thereof having a functional integrin binding site to target a vector (such as a viral, a polymeric, a cationin or a polypeptide vector) to dendritic cells. The invention also discloses a composition comprising a vector associated with lactadherin or a variant thereof.

The compositions, variants, chimeric proteins or liposomes of this invention can also be used in vitro in a method of screening and/or identifying compounds that can regulate the cross-priming of antigens and/or the phagocytosis of antigens and/or the delivery of molecules to dendritic cells. In particular, dendritic cells can be contacted with compositions, variants, chimeric proteins or liposomes of the present invention, in the presence of a candidate product or mixture, and the capacity of said candidate product or mixture to inhibit or facilitate the uptake (or binding) of said compositions can be measured.

The invention also relates to a method of screening (essentially in vitro) of compounds having the ability to induce or inhibit the expression of lactadherin. In this respect, the invention provides a method comprising (i) contacting a test compound (or mixture) with a cell culture producing a reference level of lactadherin, (ii) measuring the quantity of lactadherin produced by said cell culture, and (iii) comparing said quantity with the reference level. The cell culture can be for instance dendritic cells, preferably an established dendritic cell line. The dosage of lactadherin can be made according to conventional techniques, such as immunoassay with specific antibodies, PCR, etc. Compounds capable of increasing the expression on lactadherin may be used to stimulate the immunity of a subject, in particular the CTL response of a subject.

The invention also relates to labeled compositions or variants as described above, and their use in the monitoring of an immune response. In particular, the invention can be used to detect the presence of exosomes in a biological sample, and to correlate the level of exosomes to a physiological state of a subject. A detection method comprises for instance the collection of a sample (such as a blood sample of a subject), the contacting of said sample with a composition comprising lactadherin or a variant thereof having the ability to bind integrin, and the detection of the presence (and optionally the quantity) of exosomes bound to said lactadherin or variant thereof. The diagnostic of high levels of exosomes in a biological sample can be correlated with a pathological condition where exosomes are produced, for instance by dendritic cells (such as tumor, vital infection, etc.).

In vivo, the invention can be used to modulate an immune response, deliver a molecule to dendritic cells, modulate the phagocytosis of antigens and/or for cross-priming of antigens, and also for the monitoring of an immune response. In particular, the invention also relates to the use of lactadherin, preferably human lactadherin, or a nucleic acid encoding the same, for the stimulation of an immune response in a subject. In particular, the delivery of human lactadherin or a nucleic acid encoding the same in a subject affected with a tumor would enhance the immune response of said subject against the tumor. For this specific application, intratumoral administration would be preferred.

For in vivo applications, conventional dosages can be used and adapted by the skilled artisan. Preferably, for use in vivo, the compositions are administered by injection, such as i.v., i.a., i.p., s.c., i.m., for instance. They also can be administered by any other route. Preferably, they are formulated with appropriate acceptable vehicles, such as buffers (phosphate, potassium, chloride, etc.), isotonic solutions, saline solutions, at appropriate dosage, such as for instance between 0,1 and 10000 µg of protein by single dosage unit. The compositions may also comprise stabilizing agents (such as gels, high molecular weigh molecules, i.e., albumin, dextran, etc.), surfactants, etc.

Other aspects and advantages of the present invention will be disclosed in the following examples, which should be considered as illustrative and non limiting.

### Legend to the Figures

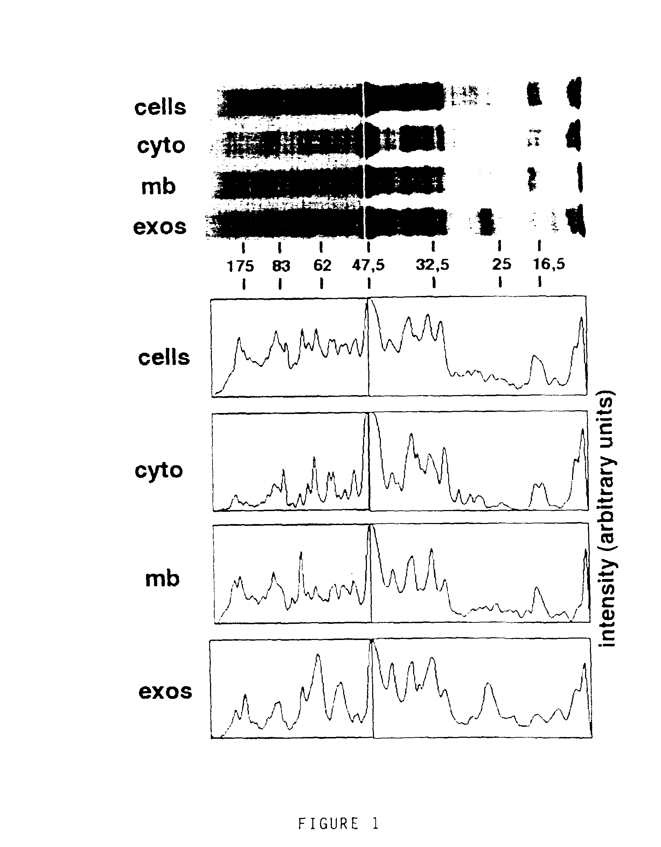

Figure 1: Overall protein composition of metabolically-labeled exosomes and D1 cells.

Exosomes purified from the supernatant of metabolically-labeled D1 cells were run on a 12% SDS gel (Exos), together with lysate (including both cytosolic and membrane components) obtained from the whole cells (Cells), or cytosol (cyto) or total membranes (mb) prepared from the same cells in the absence of detergent. The dried gel was autoradiographed for 24h (proteins bigger than 45 kDa) or 48 h (proteins smaller than 45kDa). A pic profile of each lane was obtained with the NIHImage software. Stars point to proteins abundant in the cells and absent in exosomes, dots to proteins enriched in exosomes.

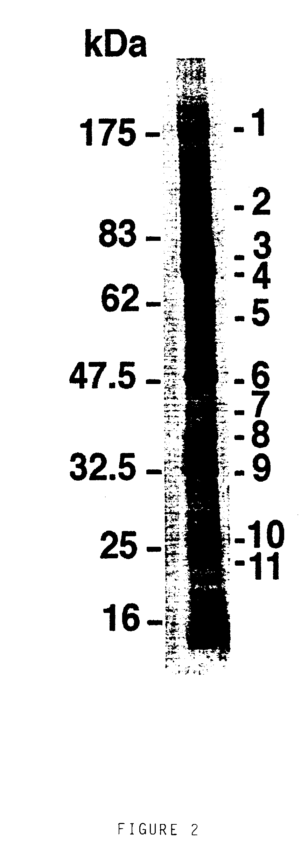

Figure 2: Protein profile of D1 exosomes as seen by coomassie blue staining.

Thirty µg of exosomes were separated on a 8-15% gradient SDS gel, and stained with coomassie brilliant blue. The band pattern is identical to that obtained with metabolically-labeled exosomes. The major bands (1 to 11) were analyzed by trypsin digestion and MALDI-TOF (see table 1)

### Materials and Methods

### Cell lines

The spleen-derived murine DC cell line D1 has been described previously (Winzler et al., 1997). It was cultured in Iscove's Modified Dulbecco's Medium (IMDM, Sigma, St Quentin, France) supplemented with 10% endotoxin-free fetal calf serum (FCS, Life Technologies, Cergy, France) and 30% R1 medium (GM-CSF-transfected NIH-3T3 fibroblasts conditioned medium). Cells were passaged twice a week in 145 mm non tissue-culture treated Petri dishes (4-5.106 cells per dish). To control their non-activated state, the level of cell surface expression of MHC class II and costimulatory molecules was checked regularly by fluorescence-activated cell scanning (FACS) as described (Winzler et al., 1997).

### Immun-electron microscopy

D1 cells, either immature or mature after treatment for 24 hours with LPS were fixed with 2% paraformaldehyde in phosphate buffer 0.2M pH 7.4 (PB) for 2 hours at room temperature. Fixed cells were processed for ultrathin sectioning and immunolabeling as described previously (Raposo et al. 1997; Liou et al., 1996). Briefly, after washing with PB and PB-50 mM glycine, cells were embedded in 7.5 % gelatine. Small blocks were infiltrated with 2.3M sucrose at 4 ᵢC for 2 hours and then frozen in liquid nitrogen. Ultrathin cryosections prepared with a Leica ultracut FCS (Wien, Austria) were retrieved with a mixture of Methycellulose and 2.3 M sucrose (vol./vol.) and indirectly immunogold labeled with the rat monoclonal anti-class II antibody M5114, followed by polyclonal antirat polyclonal antibodies (Dako, Danemark) and Protein A gold (purchased from Dr. J.W. Slot, Department of Cell Biology, Utrecht University , The Netherlands). The sections were finally contrasted and embedded in a mixture of methylcellulose and uranyl acetate and viewed under a CM120 Twin Phillips electron microscope (Eindover, The Netherlands).

### Exosomes purification

Exosomes were prepared either from the supernatant of a 3 days-old D1 culture, or from fresh culture medium incubated for 24h with 3 days-old D1 cells. We could not evidence any difference in the overall composition of exosomes purified from 3 days- or from 24h-supernatants. Exosomes were purified as previously described (Raposo et al., 1996). Briefly, the medium was cleared from cells and debris by 3 successive centrifugations at 300g (5 min), 1,200g (20 min) and 10,000g (30 min), then exosomes were recovered in the pellet after centrifugation for 1h at 70,000g. Exosomes were washed once in a large volume of PBS, centrifuged at 70,000g for 1h and resuspended in 50-200 ml of PBS with 0,01% sodium azide (NaN3). The amount of exosomal proteins recovered was measured by Bradford assay (Biorad, Ivry, France).

### Exosomes floatation on sucrose gradient

Ninety mg of exosomes were resuspended in 1ml Hepes 20mM, pH 7.2 containing 2.5M sucrose and a cocktail of protease inhibitors (see next section). A continuous 2M - 0.25M sucrose gradient was loaded on top of exosomes, and this was centrifuged for 16h at 35,000 rpm in an SW41 rotor. Fractions (1 ml each) were collected from the top of the gradient, centrifuged for 1 h at 100,000g, and the pellet was loaded on a 10% SDS gel for western blot analysis.

### Protein analysis by SDS-PAGE

Total proteins were prepared from 108 D1 cells lysed in 1 ml of Tris 50mM pH 7.5, NaCI 0.3M, TritonX-100 0.5%, NaN3 0.1%, with a cocktail of antiproteases (CLAP: chymiostatine, leupeptine, aprotinin, pepstatin, 100mM each, Sigma) for 5 min at 4ᵢC. Lysates were cleared from nuclei by centrifugation at 10,000g. To separate cytosol and total membranes, approximately 2.107 D1 cells were resuspended at 4ᵢC in 0.3 ml of Triethanolamine 10mM, EDTA 1mM, acetic acid 10 mM, sucrose 250mM titrated to pH7.4 (TEA-sucrose) supplemented with CLAP, and homogenized by 60 passages through a 25G needle. Nuclei and cell debris were cleared by centrifugation for 10 min at 1,200g. The postnuclear supernatant was centrifuged for 1h at 100,000g: cytosol was then recovered in the supernatant, and the pellet (total membranes) was resuspended in 0,3 ml of TEA-sucrose-CLAP.

### Metabolic labeling of cells and exosomes

Metabolic labeling of D1 cells was performed on 3-days-old cultures: cells were washed once in Methionine/Cysteine-free RPMI, starved for 1h at 37ᵢC in Methionine/Cysteine-free RPMI supplemented with 30% dialysed R1 medium, and incubated overnight at 37ᵢC in 20ml fresh Methionine/Cysteine-free RPMI-dialysed R1 supplemented with 5% endotoxin-free FCS and 10-20mCi/ml 35S-Methionine/Cysteine (Promix 35S, ICN, Orsay, France). Labeling medium was removed, cells were washed once in complete D1 culture medium, and further incubated for 24h in complete medium at 37ᵢC. Exosomes were purified from the supernatant; total lysates, cytosol and total membranes were prepared from the cells. Proteins (20,000cpm of each sample) were separated on a 12% SDS-PAGE, dried and autoradiographed. The pic profile of each lane was obtained using the NIHimage software.

### Protein analysis by MALDI-TOF

Thirty mg of proteins from exosomes or total lysates were run on a 6-15% SDS-PAGE, and stained with Coomassie blue (Biorad). The major bands in the exosomes preparation were excised from the gel. Briefly, proteins were digested with trypsin for 4h at 37ᵢC, and the peptide profile generated was analyzed by Matrix-Assisted Laser Desorption/lonisation - Time of Flight (MALDI-TOF, ). The peptide profile of each band was compared to those of proteins in the databases using the MS-Fit program.

### Examples

### 1. Overall protein composition of exosomes

In order to understand the molecular basis of exosome's anti-tumoral effect in vivo, we undertook an extensive characterization of the protein composition of exosomes. D1 cells were metabolically labeled overnight with 35S-Methionine/ Cysteine mix, washed once, and fresh non-radioactive medium was added to the cells for 24h. Metabolically labeled exosomes were purified from this supernatant. Out of 2 independent experiments, the amount of radioactivity recovered in exosomes was 0.25% (±0,05%) of the radioactivity incorporated into the cells. The amount of radioactivity recovered in exosomal proteins (80 cpm/mg) was similar to that recovered in proteins from the whole cells (100 cpm/mg), confirming that exosomal preparations are composed of cell-derived proteins, and are not contaminated by proteins from the extracellular medium.

The protein profile of exosomes from metabolically labeled cells was analyzed by SDS-PAGE and autoradiography and compared to that of whole D1 lysates, of cytosol or of total cellular membranes. As shown in figure 1, exosomes displayed a unique protein composition pattern. At least 7 proteins (of approximately 180, 90, 70, 58, 43, 32 and 27 kDa, see dots in figure 1, 'exos' panel) were strongly enriched in exosome preparations as compared to whole cells, cytosol or total membranes. Eight other major proteins from whole cells, cytosol or total membranes were absent or less abundant in exosomes (see stars in figure 1, 'cells' panel). Interestingly, two of the bands enriched in exosomes (70 and 58 kDa) are not detected in the cytosol or the whole D1 cells, suggesting a particularly strong mechanism of enrichment of this protein in the exosomes.

Therefore, exosomes concentrate a unique subset of cellular proteins, which most likely account for exosome's biological activities. To identify the proteins present in exosomes, thirty micrograms of exosomal proteins were loaded on a 8-15% SDS gel : the pattern of bands stained by Coomassie brilliant blue was very similar to that of metabolically labeled exosomes (compare figures 1 and 2). Therefore, all the major protein species in the exosome preparations are from cellular, and not fetal calf serum (FCS), origin. All these major bands (bands 1 to 11 in figure 2) were excised, trypsin-digested and the peptides generated were analyzed by Matrix-Assisted Laser Desorption Ionisation - Time of Flight (MALDI-TOF). The peptide profiles generated for the different bands were compared to the theoretical tryptic-peptide profiles of known proteins from the data bases. The results obtained (see Table 1 below) show that, among the identified bands, one is lactadherin (also known as Milk Fat Globule-EGF-FactorVIII, MFG-E8), a peripheral membrane-associated protein.

**Table 1**

| Band | Protein | Matching Peptide | Total Number of Peptides |
|---|---|---|---|
| 4 | Lactadherin + hsc73 | 10 11 | 28 |
| 5 | Lactadherin | 12 | 15 |

### 2. Dendritic cells but not macrophages produce Lactadherin

In view of the above evidence of the presence of lactadherin on exosomes produced by dendritic cells, dendritic cells have been analyzed to determine if they were producing lactadherin. For that purpose, an amplification reaction has been performed on total RNA prepared from dendritic cells using oligonucleotide primers specific for the mRNA of lactadherin. The sequence of the oligonucleotides is the following:

Oligo 1 (SEQ ID NO:5) : 5'-ATGCTACTCTGCGCCTCTG-3'

Oligo 2 (SEQ ID NO:6) : 5'-GTCTTGTAGCTGCTGGAGG-3'

Total RNA was prepared from 5-7x10⁶ cells by the guanidine thiocyanate method (Chomezinski and Sacchi, Annal. Biochem. 162 (1987) 156). Reverse transcription was performed in a 20µl volume:1 µg of RNA was mixed with dNTPs (250 µM each final), random hexanucleotides (2 ng, Gibco-BRL), and RNAsin (20 U, Promega) in a final volume of 15 µl, heated at 60°C for 5 minutes, and cooled down to 42°C. Four µl of 5x AMV-Reverse Transcriptase (RT) buffer and 1 µl of AMV-RT (25 U/µl, Boehringer) were added, and the reaction was performed at 42°C for 2 h. The PCR reaction was performed in a final volume of 50 µl, using 3.5 µl of the reverse transcription reaction, dNTPs (200 µM each final), MgCl2 (2 mM final), Taq or Tfl polymerase (Promega) and oligonucleotides specific for the mouse MFG-E8 cDNA (500 nM each final). The mixture was heated at 94°C for 5 minutes, submitted to 35 cycles of (94°C 30 Secondes, 53°C 30 secondes, 72°C 45 secondes), and finally to 72°C for 2 minutes. As a control, a similar PCR reaction was set up with oligonucleotides specific for the mouse actin cDNA, and submitted to 25 cycles of (94°C 30 secondes, 55°C 30 secondes and 72°C 30 secondes). Twenty µl of the PCR reactions were run on a 1% agarose gel containing ethidium bromide, and observed under UV illumination. The band amplified were of 830 bp for MFG-E8, and 800 bp for actin.

Expression of the gene coding for MFG-E8 was analyzed by RT-PCR in different murine cell lines: D1 (dendritic cell line), J774 (macrophage cell line), P815 (mastocytoma), and TS/A (mammary adenocarcinoma). D1 were used either in their immature state, or after 24 h treatment in the presence of LPS, that induces their maturation. Expression of MFG-E8 was detected only in immature D1 cells and in the TS/A mammary carcinoma: neither mature D1 cells, nor macrophages or mastocytoma expressed MFG-E8. The good quality of the cDNAs was shown by the amplification of an actin band from all the samples.

### 3. Construction of a variant of human lactadherin lacking a functional integrin binding site

This example discloses a method for the construction of variants of lactadherin lacking a functional integrin binding site. The variants obtained are essentially incapable of binding to integrin, more specifically to integrin αvβ5. Said variants therefore do not efficiently bind dendritic cells, more particularly human dendritic cells, especially human immature dendritic cells. More preferably, these variants retain the ability to bind phospholipids, in particular phosphatidyl serine. These molecules indeed represent competitors of naturally-occurring lactadherin for the binding of particulate antigens, such as apoptotic bodies or vesicles, and interfere with the development of an immune response, especially with the cross-priming of antigens.

The variants of lactadherin lacking a functional integrin binding site are constructed according to the following method.

A nucleic acid construct is provided comprising the sequence encoding human lactadherin as depicted in SEQ ID NO: 1 or at least amino acid residues 24-387 of said sequence, corresponding to the mature protein. Such a sequence is represented on SEQ ID NO:3, for instance. Obviously, variants of said sequence can also be used as the starting material. Such a construct, generally a plasmid, is replicated in a competent host cell, in order to produce suitable quantities of nucleic acid. The nucleic acid is then treated in order to render non-functional the integrin binding site of lactadherin for which it codes. In this respect, the integrin binding site lies essentially in the N-terminal part of the protein, more particularly in the amino acid residues Arg Gly Asp at position 46-48 of SEQ ID NO:1.

In a particular example, the nucleic acid is treated by deletion of all or part of the sequence encoding amino acid residues 1 to 100 of SEQ ID NO:1, said deletion rendering the protein incapable of binding integrin αvβ5. Even more preferably, the nucleic acid is treated by deletion of all or part of the sequence encoding amino acid residues 1 to 100 of SEQ ID NO:1, said deletion comprising at least all or part of the sequence encoding the amino acid residues Arg Gly Asp at position 46-48 of SEQ ID NO:1. Preferred examples of variants comprise a deletion of amino acid residues 46-48, 45-49, 40-50 or 35-55 of SEQ ID NO:1, optionally in combination with a deletion of the amino acid residues 1-23 representing the secretion signal. The deletion can be performed by various means. In particular, the deletion is performed by treatment of the nucleic acid with a restriction enzyme which cleaves said nucleic acid in the appropriate region (including at artificially created restriction sites), optionally followed by digestion of the fragments with exonucleases and/or ligation of the resulting fragments.

In another particular example, the nucleic acid is treated by mutation(s) in all or part of the sequence encoding amino acid residues 1 to 100 of SEQ ID NO:1, said mutation(s) rendering the protein incapable of binding integrin αvβ5. Even more preferably, the nucleic acid is treated by mutation(s) in all or part of the sequence encoding amino acid residues 1 to 100 of SEQ ID NO:1, said part comprising at least all or part of the sequence encoding the amino acid residues Arg Gly Asp at position 46-48 of SEQ ID NO:1. Preferred examples of variants comprise at least a mutation of one amino acid selected from the amino acid residues Arg Gly Asp at position 46-48 of SEQ ID NO:1. The mutation can be carried out by various means, including site-directed mutagenesis, amplification with mutated primers, nucleic acid synthesis and cloning, etc. The mutation can lead to the suppression of one single amino acid residue or to its replacement by another conservative or non-conservative amino acid residue.

Once they are prepared, the structure of the nucleic acids is verified by digestion and/or sequencing. The resulting nucleic acids are then expressed in a host cell, for instance a bacteria, a yeast cell or a mammalian cell, and the variants thereby produced are recovered.

Particular variants of the present invention are:
- a protein comprising amino acid residues 49-387 of SEQ ID NO:1;
- a protein of SEQ ID NO:1 with a deletion of amino acid residues 46-48;
- a protein of SEQ ID NO:1 with a deletion of amino acid residues 1-23 and 46-48;
- a protein comprising amino acids 23-387 of SEQ ID NO:1 with a mutation in amino acid residue Arg at position 46;
- a protein comprising amino acids 23-387 of SEQ ID NO:1 with a mutation in amino acid residue Gly at position 47;
- a protein comprising amino acids 23-387 of SEQ ID NO:1 with a mutation in amino acid residue Asp at position 48;

### 4. Construction of a variant of human lactadherin lacking a functional Phosphatidyl serine binding site

This example discloses a method for the construction of variants of lactadherin lacking a functional Phosphatidyl Serine (PS) binding site. The variants obtained are essentially incapable of binding to PS and therefore do not efficiently bind particulate antigens, such as apoptotic bodies or vesicles. On the other hand, said variants advantageously retain the ability of lactadherin to bind dendritic cells, more particularly human dendritic cells, especially human immature dendritic cells. Accordingly, these molecules represent competitors of naturally-occurring lactadherin for the binding of dendritic cells thereby interfering with the development of an immune response, especially with the cross-priming of antigens. These variants can also be used to deliver molecules to dendritic cells, in particular to facilitate (i.e., enhance) the uptake (e.g., the phagocytosis) of molecules by dendritic cells, in vitro, ex vivo or in vivo.

The variants of lactadherin lacking a functional PS binding site are constructed according to the following method.

A nucleic acid construct is provided comprising the sequence encoding human lactadherin as depicted in SEQ ID NO: 1, as disclosed in example 3. The nucleic acid is then treated in order to render non-functional the PS binding site of lactadherin for which it codes. In this respect, the PS binding site lies essentially in the C-terminal part of the protein, more particularly in the amino acid residues 242 to 387 of SEQ ID NO:1, even more preferably in the amino acid residues 350-387 of SEQ ID NO:1.

In a particular example, the nucleic acid is treated by deletion of all or part of the sequence encoding amino acid residues 242 to 387 of SEQ ID NO:1, more preferably comprising at least the amino acid residues 360-387 of SEQ ID NO:1, said deletion rendering the protein incapable of binding PS. The deletion can be performed by various means. In particular, the deletion is performed by treatment of the nucleic acid with a restriction enzyme which cleaves said nucleic acid in the appropriate region (including at artificially created restriction sites), optionally followed by digestion of the fragments with exonucleases and/or ligation of the resulting fragments.

In another particular example, the nucleic acid is treated by mutation(s) in all or part of the sequence encoding amino acid residues 242 to 387 of SEQ ID NO:1, more preferably at least in the sequence encoding the amino acid residues 360-387 of SEQ ID NO:1, said mutation(s) rendering the protein incapable of binding PS. The mutation can be carried out by various means, including site-directed mutagenesis, amplification with mutated primers, nucleic acid synthesis and cloning, etc.

Once they are prepared, the structure of the nucleic acids is verified by digestion and/or sequencing. The resulting nucleic acids are then expressed in a host cell, for instance a bacteria, a yeast cell, an insect cell or a mammalian cell, and the variants thereby produced are recovered.

Particular variants of the present invention are:
- a protein comprising amino acid residues 1 to 242 of SEQ ID NO:1;
- a protein comprising amino acid residues 24 to 242 of SEQ ID NO:1;
- a protein of SEQ ID NO:1 with a deletion of amino acid residues 360-387;
- a protein of SEQ ID NO:1 with a mutation in one or several amino acid residues of the sequence 360-387.

SEQUENCES

SEQ ID NO:1 : Human Lactadherin (protein)

MPRPRLLAALCGALLCAPSLLVALDICSKNPCHNGGLCEEISQEVRGDVFPSYTCTCLKG
YAGNHCETKCVEPLGMENGNIANSQIAASSVRVTFLGLQHWVPELARLNRAGMVNAWTPS
SNDDNPWIQVNLLRRMWVTGVVTQGASRLASHEYLKAFKVAYSLNGHEFDFIHDVNKKHK
EFVGNWNKNAVHVNLFETPVEAQYVRLYPTSCHTACTLRFELLGCELNGCANPLGLKNNS
IPDKQITASSSYKTWGLHLFSWNPSYARLDKQGNFNAWVAGSYGNDQWLQVDLGSSKEVT
GIITQGARNFGSVQFVASYKVAYSNDSANWTEYQDPRTGSSKIFPGNWDNHSHKKNLFET
PILARYVRILPVAWHNRIALRLELLGC

SEQ ID NO:2 : Mouse Lactadherin (protein)

MQVSRVLAALCGMLLCASGLFAASGDFCDSSLCLNGGTCLTGQDNDIYCLCPEGFTGLVC
NETERGPCSPNPCYNDAKCLVTLDTQRGDIFTEYICQCPVGYSGIHCETECSTQLGMEGG
AIADSQISASYVYMGFMGLQRWGPELARLYRTGIVNAWHASNYDSKPWIQVNLLRKMRVS
GVMTQGASRAGRAEYLKTFKVAYSLDGRKFEFIQDESGGDKEFLGNLDNNSLKVNMFNPT
LEAQYIRLYPVSCHRGCTLRFELLGCELHGCLEPLGLKNNTIPDSQMSASSSYKTWNLRA
FGWYPHLGRLDNQGKINAWTAQSNSAKEWLQVDLGTQRQVTGIITQGARDFGHIQYVESY
KVAHSDDGVQWTVYEEQGSSKVFQGNLDNNSHKKNIFEKPFMARYVRVLPVSWHNRITLR
LELLGC

SEQ ID NO: 3 : Human Lactadherin (cDNA)

agaaccccgcggggtctgagcagcccagcgtgcccattccagcgcccgcgtccccgcagc
atgccgcgcccccgcctgctggccgcgctgtgcggcgcgctgctctgcgcccccagcctc
ctcgtcgccctggatatctgttccaaaaacccctgccacaacggtggtttatgcgaggag
atttcccaagaagtgcgaggagatgtcttcccctcgtacacctgcacgtgccttaagggc
tacgcgggcaaccactgtgagacgaaatgtgtcgagccactgggcatggagaatgggaac
attgccaactcacagatcgccgcctcatctgtgcgtgtgaccttcttgggtttgcagcat

tgggtcccggagctggcccgcctgaaccgcgcaggcatggtcaatgcctggacacccagc
agcaatgacgataacccctggatccaggtgaacctgctgcggaggatgtgggtaacaggt
gtggtgacgcagggtgccagccgcttggccagtcatgagtacctgaaggccttcaaggtg
gcctacagccttaatggacacgaattcgatttcatccatgatgttaataaaaacacaag
gagtttgtgggtaactggaacaaaaacgcggtgcatgtcaacctgtttgagacccctgtg
gaggctcagtacgtgagattgtaccccacgagctgccacacggcctgcactctgcgcttt
gagctactgggctgtgagctgaacggatgcgccaatcccctgggcctgaagaataacagc
atccctgacaagcagatcacggcctccagcagctacaagacctggggcttgcatctcttc
agctggaacccctcctatgcacggctggacaagcagggcaacttcaacgcctgggttgcg
gggagctacggtaacgatcagtggctgcaggtggacctgggctcctcgaaggaggtgaca
ggcatcatcacccaggggggcccgtaactttggctctgtccagtttgtggcatcctacaag
gttgcctacagtaatgacagtgcgaactggactgagtaccaggaccccaggactggcagc
agtaagatcttccctggcaactgggacaaccactcccacaagaagaacttgtttgagacg
cccatcctggctcgctatgtgcgcatcctgcctgtagcctggcacaaccgcatcgccctg
cgcctggagctgctgggctgttagtggccacctgccacccccaggtcttcctgctttcca
tgggcccgctgcctcttggcttctcagcccctttaaatcaccatagggctggggactggg
gaaggggagggtgttcagaggcagcaccaccacacagtcacccctccctccctctttccc
accctccacctctcacgggccctgccccagcccctaagccccgtcccctaacccccagtc
ctcactgtcctgttttcttaggcactgagggatctgagtaggtctgggatggacaggaaa
gggcaaagtagggcgtgtggtttccctgcccctgtccggaccgccgatcccaggtgcgtg
tgtctctgtctctcctagcccctctcacacatcacattcccatggtggcctcaagaaa
ggcccggaagccccaggctggagataacagcctcttgcccgtcggccctgcgtcggccct
ggggtaccatgtgccacaactgctgtggcccctgtccccaagacacttccccttgtctc
cctggttgcctctcttgccccttgtcctgaagcccagcgacacagaaggggtggggcgg
gtctatggggagaaagggagcgaggtcagaggagccggcatgggttggcagggtgggcgt
ttggggccctcatgctggcttttcaccccagaggacacaggcagcttccaaatatattt
atcttcttcacggg

SEQ ID NO: 4 : Murine Lactadherin (cDNA)

gaattccgcatcagagcgcgtggacctttcccgcgtcccgcagcatgcaggtctcccgt
gtgctggccgcgctgtgcggcatgctactctgcgcctctggcctcttcgccgcgtctggt
gacttctgtgactccagcctgtgcctgaacggtggcacctgcttgacgggccaagacaat
gacatctactgcctctgccctgaaggcttcacaggccttgtgtgcaatgagactgagaga
ggaccatgctccccaaacccttgctacaatgatgccaaatgtctggtgactttggacaca
cagcgtggggacatcttcactgttctacacagctgggcatggaagggggcgccattgctg
attcacagatttccgcctcgtatgtgtatatgggtttcatgggcttgcagcgctggggcc
cggagctggctcgtctgtaccgcacagggatcgtcaatgcctggcacgccagcaactatg
atagcaagccctggatccaggtgaaccttctgcggaagatgcgggtatcaggtgtgatga
cgcagggtgccagccgtgccgggagggcggagtacctgaagaccttcaaggtggcttaca
gcctcgacggacgcaagtttgagttcatccaggatgaaagcggtggagacaaggagtttt
tgggtaacctggacaacaacagcctgaaggttaacatgttcaacccgactctggaggcac
agtacataaggctgtaccctgtttcgtgccaccgcggctgcaccctccgcttcgagctcc
tgggctgtgagttgcacggatgtctcgagcccctgggcctgaagaataacacaattcctg
acagccagatgtcagcctccagcagctacaagacatggaacctgcgtgcttttggctggt
acccccacttgggaaggctggataatcagggcaagatcaatgcctggacggctcagagca
acagtgccaaggaatggctgcaggttgacctgggcactcagaggcaagtgacaggaatca
tcacccaggggggcccgtgactttggccacatccagtatgtggagtcctacaaggtagccc
acagtgatgatggtgtgcagtggactgtatatgaggagcaaggaagcagcaaggtcttcc
agggcaacttggacaacaactcccacaagaagaacatcttcgagaaacccttcatggctc
gctacgtgcgtgtccttccagtgtcctggcataaccgcatcaccctgcgcctggagctgc
tgggctgttaatgctcagtcctgccagcccaaacgatgaggatggccagaggctgagggg
cctcctggccctgcctcccaggccctgctgccttctgtggctgacgaccttctcggcctt
ccctcctgattgtactggggctggaggcaggaagggccaggggatttcagagttgccctt
caccctttccctcaccctgcagcccccacaggcctcctgctagccccttctctcaggca
ttctgggggagttggacaggtctgagatgaatagagaagagtgaagttggggtatgt
gggctatctgtaccaaccaccccaagtcctaaacttcctgccagggcttgactcaggact
gaagggagcccctgactgcccatccctctctgcacaccacacattcctccatgttccatt
ccgggaaggagaggcccacgtccgcttgctgtcccttgggtcaccaggtcctgcctccta
tctcctgagacgcctcttgacccttgcactggagcctcagttgacaaggagactggcggg

tctggagaggtcggtggctctgggtggttgacaggttggctgtgggacctctgctggctt
gctacccaagttaacaagcagattccaaaatacattcgtgttctccactgg

## Claims

1. The use of lactadherin or a variant thereof, for the preparation of a composition for regulating the immune response.

2. The use according to claim 1, for the inhibition of the cross-priming of antigens.

3. The use according to claim 2, of a variant of lactadherin lacking a functional phospolipid binding site and/or a functional integrin binding site.

4. The use according to claim 1, for the stimulation of the phagocytosis of antigens by dendritic cells.

5. The use according to claim 1, for the stimulation of the cross-priming of antigens.

6. The use of lactadherin or a variant thereof, for the preparation of a composition for the delivery of a molecule to a dendritic cell.

7. The use according to any one of the preceding claims, wherein the lactadherin or variant thereof is human lactadherin or a variant thereof.

8. A composition comprising lactadherin or a variant thereof in association with a molecule.

9. The composition of claim 8, wherein said molecule is covalently associated to the lactadherin or variant thereof.

10. The composition of claim 8, comprising a liposome.

11. An immunogenic composition comprising lactadherin or a variant thereof in association with an antigen.

12. A variant of lactadherin, wherein said variant lacks a functional phospholipid binding site and/or a functional integrin binding site.

13. A chimeric protein comprising lactadherin or a variant thereof fused to a heterologous polypeptide.

14. A nucleic acid encoding a protein as defined in claim 13.

15. A liposome comprising a recombinant lactadherin or variant thereof.

16. A composition comprising a variant of lactadherin according to claim 12 or a chimeric protein according to claim 13 or a liposome according to claim 15.
